# EUROPEAN PATENT SPECIFICATION

(11) **EP 2 054 162 B1**
(45) Date of publication and mention of the grant of the patent: **02.11.2011**
(21) Application number: 07789292.5
(22) Date of filing: 21.08.2007
(51) Int. Cl.: B05B 11/00, B05B 11/02

(54) **DOSAGE DISPENSING CANISTER**
DOSIERSPENDBEHÄLTER
CARTOUCHE DE DÉLIVRANCE DE DOSE

(30) Priority: 26.08.2006 GB 0616918
(43) Date of publication of application: 06.05.2009
(73) Proprietor: Norwich Pharma Technologies Limited, Norwich NR4 7UH (GB)
(72) Inventor: ROSS, Calvin John, Norwich NR4 7UH (GB); SANSOM, Steven John, Norwich NR4 7UH (GB)
(74) Representative: ip21 Ltd
(86) International application number: PCT/GB2007/003198
(87) International publication number: WO 2008/025953

(56) References cited:
- EP-A- 0 311 863
- FR-A- 2 883 850
- US-A- 5 284 132
- US-B1- 6 789 750

## Description

### Field of the Invention

The invention relates to an improved form of dosage dispensing canister and is particularly applicable to multiple-dosage canisters intended to dispense narcotic drugs in spray formulations suitable for sublingual delivery.

Fentanyl and cannabis are two examples of such drugs and their formulation for inhalation, oral lozenge delivery, or a sublingual use is well known. Equally well known are a plurality of approaches to the delivery problem as such. None of these solves the dangers inherent in potent controlled drugs such as those exemplified above whose misuse can lead to undesirable and indeed life-threatening side effects.

### State of the Art as known to the applicant

Metal canisters are inherently not suitable for narcotic formulations because of the tendency of the canister in the area to corrode with time. Internal coating treatments may prove of use, but these are costly and are not immune to degradation. Metal canisters can also easily be tampered with to drain their contents because their walls are notoriously thin and this is especially the case with pressurised-propellant containing canisters.

Valves with multi polymer/metal springs to give metered dosages are available in various build options but these can require of the order of up to ten or more components. The risks of failure with repeated use are clear. Any metal components again are at risk of attack from the canister contents.

It is especially dangerous to try to package potent narcotic drugs in propellant-driven containers because any failure or puncture of the unit could automatically vent the contents into the room with dangerous and obvious inhalation risk. Furthermore, it is known that the valve assemblies in propellant-driven containers occasionally malfunction, and stick in an open position. This results in a continuous release of product, rather than in a dose-controlled fashion. The dangers of this when the product is a narcotic drug are self-evident.

Non-pressurised pumps attract the same complication objections as valve products. Separate actuators are common but these can be removed or can fall off. They may also cause the product to be sprayed in the wrong direction.

Lock out systems have been proposed by various workers but these are complex and typically incorporate electronic feedback systems, all of which again can go wrong; and whilst multiple dose packs are an attractive sale item in the (illegal) drug market, if anyone stands any reasonable chance of accessing all such pack contents then an inadvertent overdose can quickly follow.

There is finally the issue of priming which overshadows all these known proposals. Many of the proposers of aerosol and pump driven systems offer multi dose models in the belief that the products will hold prime during storage. This is not the case. All packs - in particular those incorporating dip tubes - will lose prime over time and will require up to (say) three actuations to reach the correct dose stated on the packs. The dangers are clear. Document US5284132 discloses a dispensing canister according to the preamble of claim 1.

### Summary of the Invention

The invention seeks to provide a single-dosage or multi-dosage dispensing canister, especially but not only applicable to the safe dispensing of potent narcotics, in which the risks discussed above are minimised to enable safe measured doses to be administered to patients.

Accordingly, the invention provides a one dose at a time, non-pressurised fluid dispensing canister according to claim 1.

Preferably, the surfaces comprise the respective opposite end region surfaces of a two-portion canister one of whose portions telescopes within the other. Most preferably, there are multiple engaging ratchet teeth thus allowing a single dose to be dispensed in progressive stages.

In any of these aspects, it is preferable that multiple doses can be dispensed after the first dose by imparting a twist-and-push relative movement to the canister surfaces each time a subsequent dose is desired to be dispensed. Preferably, the last such twist-and-push movement locks the canister portions. More preferably, the last such twist-and-push action either closes the orifice of the canister from which the fluid has been expelled and/or automatically ejects from the canister the orifice - containing portion thereof.

Also included within the scope of the invention is a drug dispensing canister constructed as described herein.

The embodiment of the invention to be described herein is especially suitable to sublingual delivery and is deliberately restricted to a dose pack size of two therapeutic doses only. Its design is inherently simple but leak-free and is readily portable, and its contents can be dispensed and directed with accuracy. It incorporates a mechanism which makes it impossible for the second dose to be delivered without first having exhausted the first dosage and then imparting a specific movement to the canister portions before the second dosage will be expelled at all.

The scope of the invention is defined in the claims and one particular embodiment of it will now be described in detail with reference to the accompanying drawings.

### Brief description of the drawings

In these drawings:
Figures 1 though 6 show a two-dosage container, embodying the invention, in conventional serial orthogonal projectional views in which figure 4 is a section along the line a-a of figure 3;
Figures 7 through 12 show similarly conventionally drawn views of a second, one-dosage-only, embodiment of the invention in which figures 10 and 11 are respectively sections along the line A-A of figure 9 and the line c-c of figure 12; and
Figure 13 is a scrap section detail showing the one-way ratchet mechanism used in either of these embodiments.

### Description of the preferred embodiments

Both embodiments consist of non-metallic materials and comprise essentially a circular-cylindrical elongated canister whose two half-portions telescope. These canisters are relatively small in size so as to be readily pocketed. Each contains a measured dosage of potent narcotic in fluid form and each is initially sheathed in suitable tamper-evident collar shields which must be removed before any dosage can be dispensed from the canister.

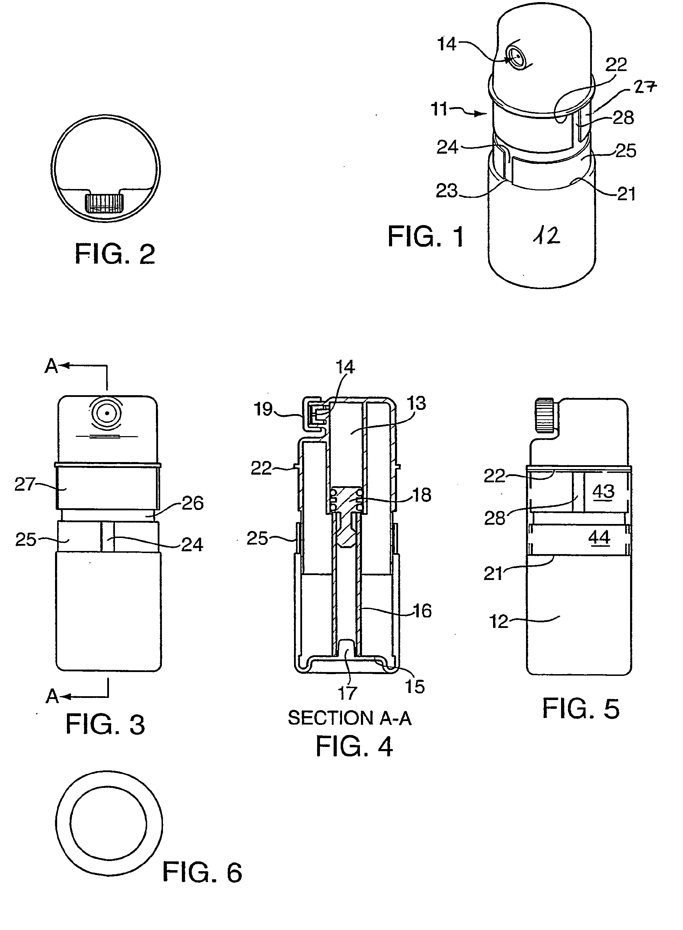

In the two-dosage canister of the figures 1 through 6 the circular-cylindrical top portion 11 of the canister telescopes within the similarly circular-cylindrical bottom portion 12. The top portion 11 as illustrated in figure 4 incorporates a circular-cylindrical chamber 13 into which the narcotic in fluid or powder form is packed. An orifice 14 is positioned adjacent the closed upper end of the top portion 11 of the canister and the canister contents are sprayed through this orifice to the atmosphere via a nozzle when the canister is activated.

The close end face 15 of the bottom portion of the canister is dished, as shown, and locates into an abuts one end of a tubular cylindrical piston rod 16. A spigot 17 is a push fit inside the end of the tubular piston rod 16 and locates the rod centrally on the recessed dished surface 15 of which the spigot 17 forms an integral protrusion.

The other end of the tubular piston rod 16 locates a piston 18 which is sized, and sealed to be a sliding fluid-tight push fit inside the chamber 13. The piston 18 when activated therefore acts to expel progressively the narcotic from the chamber 13 out via the orifice and nozzle 14.

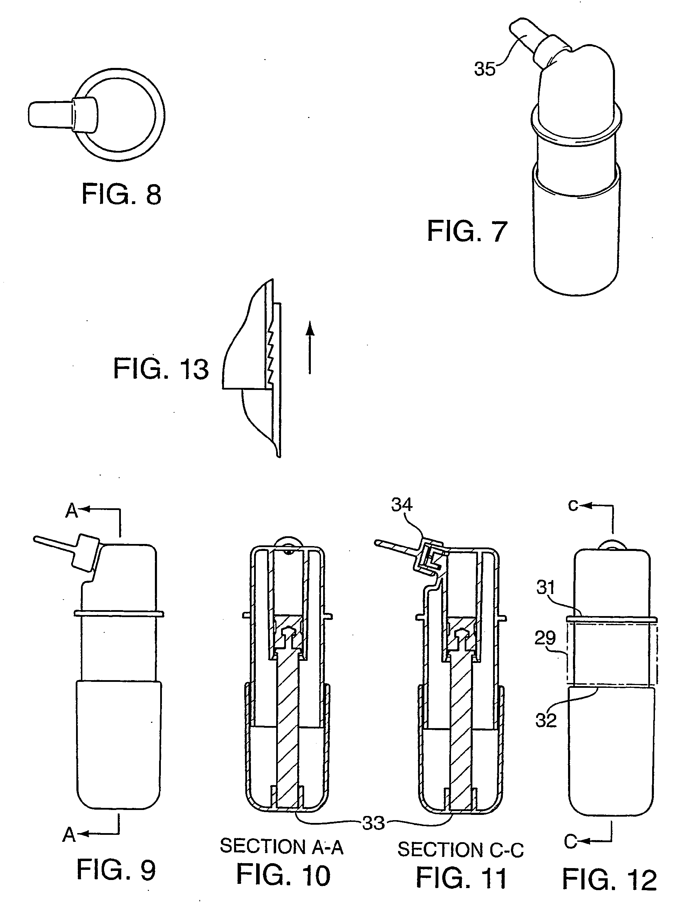

As initially supplied, the orifice and its nozzle are protected by a removable cap 19 and the gap between the radially outermost surface 21 of the canister base portion 12 and the radially outermost surface 22 of the top portion 11 is covered by 2 peelable plastics transparent tamper-evident sealing rings 43, 44 (shown in chain line in figure 5 that removed from each other figure). With the canister contents already packed into the chamber 13 and the piston and piston rod assembly in place, a one-way ratchet mechanism of the kind shown in figure 13 constrains the canister halves subsequent to the two halves being assembled.

The radially outermost surface 21 of the bottom canister half 12 is formed with an inward-projecting key tab 23. This key is a sliding engagement fit in a guide groove 24 formed along the length of a ring 25 which is bonded to the surface of the canister half 11. The key 23 is of restricted longitudinal depth such that, as the two halves of the canister are brought towards one another by pushing the end face of one half towards the other, the key tab 23, when it has cleared the groove 24 axially can then move radially along a space 26 defined between the end of the ring 25 and the protruding portion 27 of the canister top half 11.

That protruding portion is labelled 27 in figure 3 of the drawings and it incorporates, as figures 1 and 5 each show, its own longitudinal groove 28 along which the key can again move when properly positioned to enter the groove 28.

For the key 23 to make these successive movements, it must first be sent along the groove 24 by virtue of the bottom canister half 12 being pushed towards the top canister half 11 I as outlined above. The bottom half 12 must then be twisted radially about the top half 11 I by 45° to move the key 23 along the defined space 26 and into alignment with another groove 28. Another axial push on the inface in of the bottom half of canister 12 sends the key 23 along the groove 28 until the radially outermost surface 21 of the canister half 12 is up against the underside of the radially outermost surface 22 of the top canister half 11.

These two successive movements will of course actuate the piston 18 to expel sequentially two successive doses of fluid from the chamber 13 via the spray-nozzle-containing orifice 14. The whole design of the canister makes it impossible for the second dosage to be dispensed before the first has been fully dispensed and also prevents the second dosage from being dispensed until after the twist-and-push action has been applied to the bottom canister half 12.

The one-way ratchet construction has the dual function, firstly of resisting any attempt to pull the two canister halves apart once they have been initially assembled and secondly of effectively locking the canister shut once both doses have been fully dispensed. The two circular-cylindrical canister halves are sufficiently resilient to allow the ratchet teeth to ride over one another in axial relative movement whilst being tough enough to resist at least initial attempts to puncture or break open the canister surfaces.

The protective cap 19 must of course be removed before any dose can be dispensed. As well as having a protective function to prevent dirt entering the nozzle, in preferred embodiments of the device, the cap is a sealing cap to prevent moisture ingress into the device, and to act as an additional safety feature in the unlikely event of leakage of the contents through the valve. The tamper-evident transparent plastics sealing rings 43 and 44 ideally should be removed in sequence 44 followed by 43, 44 alone being removed to dispense the first dosage and then 23 subsequently being peeled away when the second dosage is ready for dispensation.

The embodiment of figures 8 through 12 differs principally from the one described above in that it is a one-dosage-only dispenser. The tamper sealing cover 29 of this embodiment occupies the whole of the gap between the radially outermost surface 31 of the top canister half and the radially outermost surface 32 of the bottom half. When the tamper seal 29 is broken and peeled off, a single push on the end face 33 of the canister along the central axis thereof dispenses the whole dose in one go.

Other differences seen in the embodiment are, firstly, that the removable cap 34 has a bayonet-style extension 35 and is itself a push fit, not a screw fit, on the dispensing orifice; and the cap is angled at approximately 75° to the central longitudinal axis of the canister, as is the dispensing orifice and nozzle so that an accurately directed dosage spray can be dispensed.

This latter feature is especially useful if the spray is to be sublingually directed and the canister is to be inverted (from the position as shown in the drawings) in order to deliver it.

The scope of the invention is defined in the claims that follow.

## Claims

1. A one dose at a time, non-pressurised fluid dispensing canister (11,12), comprising a first portion (13) and a second portion (14) of unequal circumferences, each portion with an end region surface (5), from which canister, in use, fluid is dispensed by the user telescoping one portion into the other, and **characterised in that** the telescoped canister portions incorporate a one-way ratchet mechanism with multiple engaging teeth which mechanism, once the portions are assembled to form the canister, resists any subsequent attempt to disassemble the first and second portion and which ratchet mechanism once a first dose has been dispensed, acts to prevent any subsequent attempted movement of the said surfaces into or towards their non-dispensing starting position.

2. A canister according to claim 1 **characterised in that** multiple doses can be dispensed after the first dose by imparting a twist-and-push relative movement to the canister surfaces [11-12] each time a subsequent dose is desired to be dispensed.

3. A canister according to claim 2 **characterised in that** the last such twist-and-push movement locks the canister portions.

4. A canister according to claim 2 or claim 3 **characterised in that** the last such twist-and-push movement closes the orifice [14] of the canister from which the fluid has been expelled.

5. A canister according to any of claims 2, 3 and 4 **characterised in that** the last such twist-and-push movement automatically ejects from the canister the portion thereof containing the orifice from which the fluid has been expelled.

6. A canister according to any of the claims 2 to 5 **characterised in that** the canister portions [11, 12] push and twist about one common axis [17].

7. A canister according to any preceding claim **characterised in that** the mechanism acting to prevent movement of the canister surfaces towards their non-dispensing starting position acts in line with the telescoping action of the canister portions [11,12]

8. A canister according to any preceding claim **characterised in that** the mechanism acting to prevent any attempted movement of the canister surfaces into the non-dispensing starting position operates without fracturing any internal component of the canister

9. A canister according to any preceding claim **characterised in that** the mechanism imparting a twist-and-push relative movement to the canister surfaces each time a subsequent dose is desired to be dispensed, operates without fracturing any internal component of the canister.

10. A canister according to any preceding claim **characterised in that** there is a band [23, 24] around the outside surface of the canisters, said band being removable in order to allow the canister portions to telescope and allow fluid to be dispensed.

11. A canister according to either of claims 8 and 9 **characterised in that** the band forms a tamper-evident seal on the canister.

12. A canister according to any preceding claim **characterised in that** the telescoping canister portions are each of substantially constant width along their respective lengths.

13. A canister according to any preceding claim **characterised in that** fluid is discharged at an angle to the axis along which the canister portions [11, 12] telescope.

14. A canister according to claim 13 **characterised in that** the angle is substantially a right angle.

15. A canister according to claim 13 **characterised in that** the angle is an acute angle when measured with reference to the fluid-dispensing movement of the telescoping canister portions [11,12].

16. A canister according to claim 15 **characterised in that** the acute angle is approximately 75°.

## Patentansprüche

1. Ein jeweils eine Dosis Fluid spendender, nicht unter Druck gesetzter Behälter [11, 12], bestehend aus einem ersten Teil [13] und einem zweiten Teil [14] mit ungleichen Umfängen, wobei jeder Teil eine Endbereichsfläche [5] besitzt, und wobei aus diesem Behälter bei Gebrauch Fluid abgegeben wird, indem der Benutzer einen Teil in den anderen schiebt, und der **dadurch gekennzeichnet ist, dass** die teleskopierbaren Behälterteile einen Ein-Weg-Sperrmechanismus mit mehreren ineinandergreifenden Zähnen enthalten, wobei dieser Mechanismus nach Zusammenbau der Teile zum Behälter allen nachfolgenden Versuchen zum Auseinandernehmen des ersten und zweiten Teils widersteht und dieser Sperrmechanismus nach Abgabe einer ersten Dosis alle nachfolgend unternommenen Versuche zur Bewegung der genannten Flächen in ihre oder in Richtung ihrer nicht-spendenden Ausgangsposition verhindert.

2. Ein Behälter nach Anspruch 1, **dadurch gekennzeichnet, dass** mehrere Dosen nach der ersten Dosis abgegeben werden können, indem jedes Mal, wenn eine weitere Dosis abgegeben werden soll, eine relativ zu den Behälterflächen [11-12] erfolgende Dreh- und Drückbewegung ausgeübt wird.

3. Ein Behälter nach Anspruch 2, **dadurch gekennzeichnet, dass** die letzte derartige Dreh- und Drückbewegung die Behälterteile arretiert.

4. Ein Behälter nach Anspruch 2 oder Anspruch 3, **dadurch gekennzeichnet, dass** die letzte derartige Dreh- und Drückbewegung die Öffnung [14] des Behälters schließt, aus der das Fluid ausgestoßen wurde.

5. Ein Behälter nach einem oder mehreren der Ansprüche 2, 3 und 4, **dadurch gekennzeichnet, dass** die letzte derartige Dreh- und Drückbewegung automatisch den Teil aus dem Behälter auswirft, der die Öffnung enthält, aus der das Fluid ausgestoßen wurde.

6. Ein Behälter nach einem oder mehreren der Ansprüche 2 bis 5, **dadurch gekennzeichnet, dass** die Behälterteile [11, 12] sich um eine gemeinsame Achse [17] drehen und drücken lassen.

7. Ein Behälter nach einem oder mehreren der vorherigen Ansprüche, **dadurch gekennzeichnet, dass** der Mechanismus zur Verhinderung einer Bewegung der Behälterflächen in Richtung ihrer nicht-spendenden Ausgangsposition im Einklang mit der Teleskopierung der Behälterteile [11, 12] funktioniert.

8. Ein Behälter nach einem oder mehreren der vorherigen Ansprüche, **dadurch gekennzeichnet, dass** der Mechanismus zur Verhinderung von versuchten Bewegungen der Behälterflächen in die nicht-spendende Ausgangsposition funktioniert, ohne einen Bruch der inneren Bestandteile des Behälters zu verursachen.

9. Ein Behälter nach einem oder mehreren der vorherigen Ansprüche, **dadurch gekennzeichnet, dass** der Mechanismus zur Ausübung einer relativ zu den Behälterflächen erfolgenden Dreh- und Drückbewegung bei jedem Mal, wenn eine weitere Dosis abgegeben werden soll, funktioniert, ohne einen Bruch der inneren Bestandteile des Behälters zu verursachen.

10. Ein Behälter nach einem oder mehreren der vorherigen Ansprüche, **dadurch gekennzeichnet, dass** ein Band [23, 24] um die Außenfläche des Behälters herum vorhanden ist, wobei dieses Band entfernt werden kann, damit die Behälterteile teleskopiert werden können und Fluid abgegeben werden kann.

11. Ein Behälter nach einem der beiden Ansprüche 8 und 9, **dadurch gekennzeichnet, dass** das Band einen Originalitätsverschluss des Behälters darstellt.

12. Ein Behälter nach einem oder mehreren der vorherigen Ansprüche, **dadurch gekennzeichnet, dass** die teleskopierbaren Teile des Behälters im Wesentlichen über ihre jeweilige Länge hinweg eine konstante Breite aufweisen.

13. Ein Behälter nach einem oder mehreren der vorherigen Ansprüche, **dadurch gekennzeichnet, dass** die Fluidabgabe in einem Winkel zur Achse erfolgt, entlang der die Behälterteile [11, 12] teleskopiert werden.

14. Ein Behälter nach Anspruch 13, **dadurch gekennzeichnet, dass** der Winkel im Wesentlichen ein rechter Winkel ist.

15. Ein Behälter nach Anspruch 13, **dadurch gekennzeichnet, dass** der Winkel ein spitzer Winkel ist, wenn er in Bezug auf die Fluid spendende Bewegung der teleskopierbaren Behälterteile [11, 12] gemessen wird.

16. Ein Behälter nach Anspruch 15, **dadurch gekennzeichnet, dass** der spitze Winkel ungefähr 75° beträgt.

## Revendications

1. Une cartouche de délivrance de fluide non pressurisé, à raison d'une dose à la fois(11, 12), comprenant une première section (13) et une seconde section de circonférences inégales, chaque section présentant une surface d'extrémité (5) à partir de laquelle, en utilisation, le fluide de la cartoucheest délivré par l'utilisateur par télescopage d'une sectionavec l'autre, et **caractérisée en ce que** les sections de cartouche télescopées intègrent un mécanisme de cliquet à sens unique et à dents égrenées multiples, lequel mécanisme, une fois que les sections sont assemblées pour former la cartouche, résiste à toute tentative subséquente de désassemblage de la première et la seconde section, et lequel mécanisme de cliquet, une fois que la première dose a été délivrée, empêche toute nouvelle tentative de mouvement desdites surfaces vers leur position de départ, dans laquelle le fluide n'est pas délivré.

2. Une cartouche selon la revendication 1, **caractérisée en ce que** des doses multiples peuvent être délivrées après la première dose en transmettant un mouvement relatif de pression-rotation aux surfaces de la cartouche [11-12] à chaque fois qu'une nouvelle dose doit être délivrée.

3. Une cartouche selon la revendication 2, **caractérisée en ce que** ce dernier mouvement de pression-rotation verrouille les sections de la cartouche.

4. Une cartouche selon la revendication 2 ou 3, **caractérisée en ce que** ce dernier mouvement de pression-rotation ferme l'orifice [14] de la cartouche à partir duquel le fluide a été expulsé.

5. Une cartouche selon l'une quelconque des revendications2, 3 et 4, **caractérisée en ce que** ce dernier mouvement de pression-rotation éjecte automatiquement de la cartouche la sectionde celle-ci contenant l'orifice à partir duquel le fluide a été expulsé.

6. Une cartouche selon l'une quelconque des revendications 2 à 5, **caractérisée en ce que** les mouvements de pression-rotation des sections de cartouche [11, 12] s'effectuent autour d'un axe commun [17].

7. Une cartouche selon l'une quelconque des revendications précédentes, **caractérisée en ce que** le mécanisme permettant d'empêcher le mouvement des surfaces de la cartouche vers leur position de départ, dans laquelle le fluide n'est pas délivré, agit dans le prolongement de l'action de télescopage des sections de la cartouche [11,12].

8. Une cartouche selon l'une quelconque des revendications précédentes, **caractérisée en ce que** le mécanisme permettant d'empêcher toute tentative de mouvement des surfaces de la cartouche vers la position de départ, dans laquelle le fluide n'est pas délivré, fonctionne sans fracturer aucune des pièces internes de la cartouche.

9. Une cartouche selon l'une quelconque des revendications précédentes, **caractérisée en ce que** le mécanisme permettant de transmettre un mouvement relatif de pression-rotation aux surfaces de la cartouche à chaque fois qu'une nouvelle dose doit être délivrée fonctionne sans fracturer aucune des pièces internes de la cartouche.

10. Une cartouche selon l'une quelconque des revendications précédentes, **caractérisée en ce qu'**une bande [23,24] entoure la surface externe des cartouches, ladite bande pouvant être retirée de façon à permettre le télescopage des sections de la cartouche et la délivrance du fluide.

11. Une cartouche selon les revendications 8 ou 9, **caractérisée en ce que** la bande forme un dispositif de scellement avec indicateur d'effraction sur la cartouche.

12. Une cartouche selon l'une quelconque des revendications précédentes, **caractérisée en ce que** les sections de cartouche télescopées présentent chacune une largeur substantiellement constante le long de leurs longueurs respectives.

13. Une cartouche selon l'une quelconque des revendications précédentes, **caractérisée en ce que** le fluide est expulsé en formant un angle par rapport à l'axe de télescopage des sections de la cartouche [11, 12].

14. Une cartouche selon la revendication 13, **caractérisée en ce que** l'angle est substantiellement un angle droit.

15. Une cartouche selon la revendication 13, **caractérisée en ce que** l'angle est un angle aigu lorsqu'il est mesuré par rapport au mouvement de délivrance du fluide effectué par les sections de cartouche télescopées [11, 12].

16. Une cartouche selon la revendication 15, **caractérisée en ce que** l'angle aigu est d'environ 75°.
